# EUROPEAN PATENT SPECIFICATION

(11) **EP 4 114 840 B1**
(45) Date of publication and mention of the grant of the patent: **25.06.2025**
(21) Application number: 21709768.2
(22) Date of filing: 24.02.2021
(51) Int. Cl.: A61K 31/7135, A61P 31/04

(54) **ANTIBIOTICS**
ANTIBIOTIKA
ANTIBIOTIQUES

(30) Priority: 06.03.2020 GB 202003320
(43) Date of publication of application: 11.01.2023
(73) Proprietor: Aston University, Birmingham West Midlands B4 7ET (GB)
(72) Inventor: COX, Jonathan, Birmingham West Midlands B4 7ET (GB); BARRY, Nicolas, Bradford BD7 1DP (GB); HARRISON, James, Birmingham West Midlands B4 7ET (GB); NOLAN, Victoria, Birmingham West Midlands B4 7ET (GB); PITTO-BARRY, Anaïs, Bradford BD7 1DP (GB); SOLDEVILA-BARREDA, Joan, Bradford BD7 1DP (GB)
(74) Representative: Henderson, Helen Lee
(86) International application number: PCT/GB2021/050471
(87) International publication number: WO 2021/176199

(56) References cited:
- WO-A2-2013/033018
- WO-A2-2016/087932
- CN-A- 110 256 502
- SOLDEVILA-BARREDA JOAN J. ET AL: "Synthesis, Characterisation and In Vitro Anticancer Activity of Catalytically Active Indole-Based Half-Sandwich Complexes", MOLECULES, vol. 25, no. 19, 3 October 2020 (2020-10-03), DE, pages 4540, XP093062649, ISSN: 1433-1373, DOI: 10.3390/molecules25194540
- BASHARI V ET AL: "Synthesis, characterization, and antimicrobial activity of organometallic ruthenium(II) complexes", JOURNAL OF UNDERGRADUATE CHEMISTRY RESEARCH, vol. 5, 1 January 2006 (2006-01-01), pages 99 - 104, XP055795667
- BASHARI ET AL: "SYNTHESIS, CHARACTERIZATION, AND ANTIMICROBIAL ACTIVITY OF ORGANOMETALLIC RUTHENIUM(II) COMPLEXES", JOURNAL OF UNDERGRADUATE CHEMISTRY RESEARCH, vol. 5, no. 2, 2006, pages 99 - 104, XP009526966
- NEUMANN BERND ET AL: "Synthesis, Structures and Comparison of Neutral Complexes Formed by2-(2'-Pyridyl)indole and d6 Transition Metals", EUROPEAN JOURNAL OF INORGANIC CHEMISTRY, vol. 2007, no. 3, 1 January 2007 (2007-01-01), DE, pages 472 - 480, XP055795498, ISSN: 1434-1948, DOI: 10.1002/ejic.200600730
- GANESAN VINOTHKUMAR ET AL: "Correlation between the Structure and Catalytic Activity of [Cp*Rh(Substituted Bipyridine)] Complexes for NADH Regeneration", INORGANIC CHEMISTRY, vol. 56, no. 3, 10 January 2017 (2017-01-10), Easton , US, pages 1366 - 1374, XP055791251, ISSN: 0020-1669, Retrieved from the Internet <URL:https://pubs.acs.org/doi/pdf/10.1021/acs.inorgchem.6b02474> DOI: 10.1021/acs.inorgchem.6b02474
- CÔRTE-REAL LEONOR ET AL: "The key role of coligands in novel ruthenium(II)-cyclopentadienyl bipyridine derivatives: Ranging from non-cytotoxic to highly cytotoxic compounds", JOURNAL OF INORGANIC BIOCHEMISTRY, ELSEVIER INC, US, vol. 150, 20 June 2015 (2015-06-20), pages 148 - 159, XP029322862, ISSN: 0162-0134, DOI: 10.1016/J.JINORGBIO.2015.06.015
- JAMES D. BLAKEMORE ET AL: "Half-Sandwich Iridium Complexes for Homogeneous Water-Oxidation Catalysis", JOURNAL OF THE AMERICAN CHEMICAL SOCIETY, vol. 132, no. 45, 17 November 2010 (2010-11-17), pages 16017 - 16029, XP055065578, ISSN: 0002-7863, DOI: 10.1021/ja104775j

## Description

The invention relates to organometallic compounds for use as antibiotics, for the treatment of bacterial infections.

There is currently an urgent need for new antibiotics and strategies for treating drugresistant bacterial infections. Bacterial resistance has developed against many, if not all, of current antibiotics. In the meantime, few antibiotics have being developed over the last number of decades. A primary cause of drug resistance is the over-use of antibiotics that can result in alteration of microbial permeability, alteration of drug target binding sites, induction of enzymes that destroy antibiotics (such as β-lactamases) and induction of efflux mechanisms.

The need for new antibiotics is even more urgent in the case of gram-negative bacteria such as *Pseudomonas, Klebsiella* and other bacteria such as *Escherichia* and *Acinetobacter.*

Many of the currently known antibiotics are structurally related to one another, and therefore are targets for drug resistance in bacteria. The inventors have unexpectedly identified a new class of organometallic compounds which have been found to have antibacterial properties.

Bashari V et al, J, Undergrad. Chem. Res, 5 (2006), 99-104) describes the testing of a variety of different ruthenium II complexes against Gram positive and Gram negative bacteria. The compounds include a number of complex pyridine-containing multi ring structures, but no indole-containing structures. Neumann B. et al., Eur. J. Inorg. Chem. 2007(3), 472-480 describes the production (1-amido-4-imine) metallacycles. There is no suggestion of any anti-bacterial activity for such compounds. Soldevila-Barreda J et al, Molecules, 25 (3) (3 October 2020), 4550 was published after the priority date of the invention and discloses indole based half-sandwich complexes.

The invention provides a compound of formula I for use in the prophylaxis or treatment of a bacterial infection as defined in the claims.

Components A and B are bound to the transition metal M through the nitrogen atom of the indole or pyridine and are additionally bound to one another by a bond, such as a carbon-carbon bond. That bond is typically between the carbons of each of A and B immediately adjacent to the nitrogen atom of the indole or pyridine. That is, carbon 2 or 6 of the pyridine or carbon number 2 of the indole group.

The indoles or pyridines may be functionalised with one or more substituents which will influence the lipophilicity of the resulting organometallic compound (e.g. sulfonic acid, benzene), have an effect on the electronic properties of the complexes (e.g. electrondeficient ligands such as carboranes), or bio-active ligands (e.g. sugars).

L may comprise one or two aromatic rings. The rings may be linked via a carbon-carbon bond for example, or may share a common bond. L may be a substituted or non-substituted cyclopentadienyl or a substituted or non-substituted benzene ring. Functionalisation of the ligand L may be performed to modify the chemical and physical properties of the resulting complexes, for example by introducing one or more side groups selected from a sugar, hydroxyl, halogen, carboxylate, amide, triazole or a C1 - C4 straight or branched chain alkyl group. The halogen may be selected from, for example, chlorine, fluorine, bromine or iodine.

One or more active targeting moieties against bacteria may be connected to the aromatic ring via, for example, a carboxylate acid, amide or triazole group. The substitutions may be used to adjust the activity of the compound against the bacteria, alternatively, for example, adjust the solubility of the compound to allow the mode of administration to a subject to be optimised. One, two, three, four, five or all of the carbon atoms of the aromatic ring may be substituted.

L, A or B may be substituted by, for example, one or more methyl or -CH (CH₃)₂.

L may comprise a C5 or C₆ aromatic ring.

L may be selected from para-cymene, pentamethylcyclopentadiene, hexamethyl benzene, biphenyl and cyclopentadienyl.

M is typically selected from Ru^{II}, Rh^{III}, Ir^{III} and Os^{II}.

Z is typically selected from a halogen or coordinating ligand compound wherein Z is preferably selected from Cl, Br, I, water, methanol, dimethyl sulfoxide, acetonitrile and dimethylformamide.

More preferably, the compound is selected from FKB3, FKB10 and FKB11.

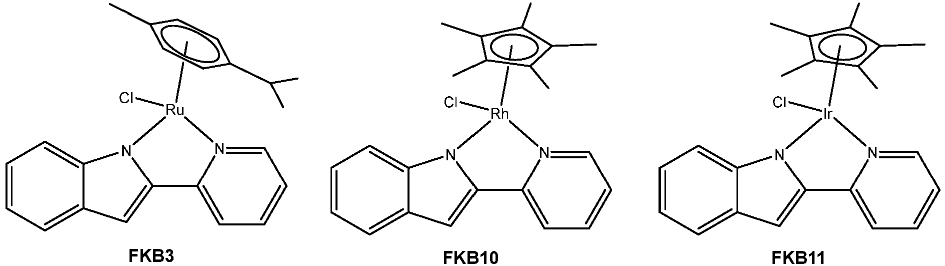

The chlorine atom of the compound may be replaced by one or more alternative halogen or coordinating ligands as defined above.

The compounds and pharmaceutically acceptable salts may be used in combination with the pharmaceutically acceptable carrier. The carrier may, for example, be a solvent, such as an aqueous solvent, or alternatively a filler or bulking agent such as talc or carboxymethylcellulose.

The compound of pharmaceutically acceptable salt may be in a form suitable to be administered to a subject. These include, for example, oral tablets, oral suspensions, oral solutions, injectable formulations, such as intra-venously, intra-muscularly or intraperitonealy, as eye drops or topical lotions or creams. The formulation may also be in a form suitable for administration through the nose or lungs, such as in the form of an aerosol.

Oral administration of compounds can be enclosed in hard or soft shell gelatine capsules, compressed into tablets or incorporated directly into the food of a patient's diet. Compounds can also be combined with one or more excipients and be used in the form of ingestible tablets, buccal tablets, troches, capsules, elixirs, suspensions, syrups, wafers and the like. Such compounds and preparations will typically contain at least 0.1% wt of active compound. The percentage of these compositions and preparations may vary depending on a given dosage form. Binders such as gum, tragacanth, acacia, corn starch or gelatine may be used. Excipients such as dicalcium phosphate, a disintegrating agent such as corn starch or potato starch, or a lubricant such as magnesium stearate may be used. Sweetening agents such as sugars or aspartame, or flavouring agents such as peppermint oil may be used. Additionally, when the dosage form is in the form of a capsule, a liquid carrier such as a vegetable oil or a polyethylene glycol may be used. Preservatives such as methyl and propyl parabens may be used together with one or more dyes or coatings, such as gelatine, wax, shellac and sugar or the like.

The subject to be treated may be a mammal, such as a human, but may also include cats, dogs, pigs, sheep, cows or rodents such as rats or mice.

A bacterial infection may be caused by a Mycobacterium, a Gram-positive bacterium or a Gram-negative bacterium, most typically a Gram-negative bacterium. The bacterium may be antibiotic resistant, for example by producing a beta-lactamase or carbapenumase.

Bacteria may be selected from the genus *Mycobacterium, Escherichia, Salmonella, Clostridium, Streptococcus, Staphylococcus, Pseudomonas, Klebsiella, Acinetobacter,* and *Enterobacter,* most preferably the bacterium is selected from *Mycobacterium abcsessus, Escherichia coli, Pseudomonas aeruginosa* or *Klebsiella pneumonia.*

The invention will now be described by way of example only with reference to the following figures:
FIGURE 1 provides stability of FKB10 in MeOD (A) and MeOD/D₂O (B), FKB11 in MeOD (C) and MeOD/D₂O (D) and FKB3 in MeOD (E) and MeOD/D₂O (F);
FIGURE 2 start point and end point data for compounds tested against M. abscessus measured at 570 nm. A FKB3, B FKB10, C FKB11;
FIGURE 3 start point and end point data for compounds tested against E.coli ATCC measured at 570 nm. A FKB3, FKB10, C FKB11;

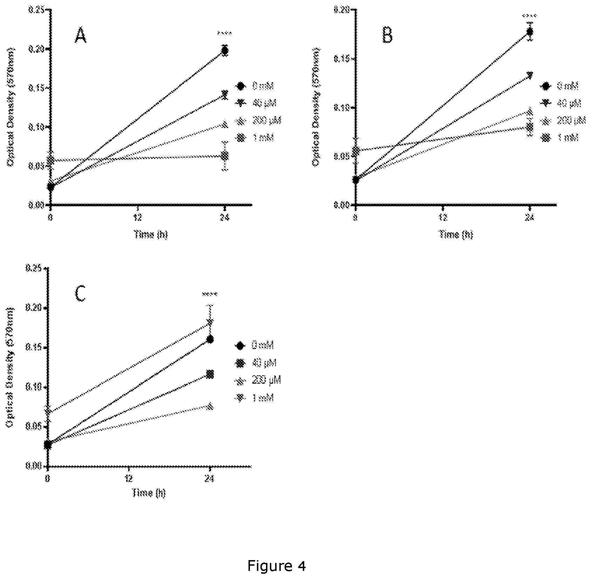

FIGURE 4 start point and end point data for compounds tested against E.coli J53 2138E measured at 570 nm. A FKB3, FKB10, C FKB11,;
FIGURE 5 start point and end point data for compounds tested against E-coli J53 2140E measured at 570 nm. A FKB3, B FKB10, C FKB11;
FIGURE 6 start point and end point data for compounds tested against E.coli 1496 ESBL measured at 570 nm. A FKB3 B FKB10, C FKB11;
FIGURE 7 start point and end point data for compounds tested against P. aeruginosa ATCC measured at 570 nm. A FKB3, B FKB10, C FKB11;
FIGURE 8 start point and end point data for compounds tested against K. pneumoniae H467 measured at 570 nm. A FKB3, B FKB10, C FKB11.

### 1.1 Synthesis and stability of FKB3, FKB10 and FKB11

**FKB3:** p-cymene ruthenium dimer (118.2 mg, 0.19 mmol) and 2-(2-pyrindyl)-1H-Indole (75 mg, 0.38 mmol) were placed in a 100 mL 2 neck round bottom flask and dissolved in 15 ml dichloromethane. Triethylamine (54 µl, 0.38) was added to the reaction mixture. The dark orange solution was then left stirring overnight at room temperature under nitrogen. The solvent was removed under vacuum. The orange crude was dissolved in ethyl acetate and washed with 0.1 M HCl (3 x 10 mL) and brine (1 x 10 mL). The combined organic layer was dried over MgSO₄, filtered and dried over vacuum to obtain an orange powder. The product was purified by chromatography (acetone/dichloromethane 10:90 v/v). Yield: 72.1 mg (40.2 %). HRMS-ESI+: calculated (M-Cl)⁺ 429.0905 m/z, found 429.0906 m/z. 1H NMR (400MHz, CDCl₃): δ_{H} 8.94 ppm (1H, d, J = 5.8 Hz), 7.73 (1H, d, J = 8.2 Hz), 7.68 (1H, d, J = 7.0 Hz), 7.63 (1 H, d, J = 8.2 Hz), 7.52 (1H, d, J = 8.2 Hz), 7.15 (2H, t, J = 7.5 Hz), 7.01 (3H, m), 5.95 (1H, d, J = 6.1 Hz), 5.59 (1H, d, J = 6.1 Hz), 5.53 (15 H, s), 2.35 (1 H, sept, J = 6.9 Hz), 2.31v(3H, s), 0.88 (3 H, d, J = 6.9 Hz), 0.86 (3H, d, J = 6.9 Hz).

**FKB10:** Rh dimer [(Cp*)RhCl₂]₂ (110 mg, 0.18 mmol) and 2-(2-pyrindyl)-1H-Indole (78 mg, 0.38 mmol) were placed in a 2 neck round bottom flask and dissolved in 25 mL of dichloromethane to obtain an orange solution. Triethylamine (66 µL, 0.47 mmol) was added and the reaction mixture left to react for 24 h at room temperature. The solvent of the reaction mixture was removed under vacuum and the orange solid dissolved in dichloromethane (20 mL) and extracted with 0.1 M HCl solution (1 x 10 mL). The combined organic layer was dried over magnesium sulfate, filtered and dried over vacuum to obtain an orange powder. The product was purified by column chromatography in silica, acetone / dichloromethane (1:9 v/v) and recrystallised in dichloromethane to obtain crystalline orange needles. Yield: 63.4 mg (38.1 %). HRMS-ESI+: calculated (M-Cl)⁺ 431.1000 m/z, found 431.0833 m/z. 1H NMR (400MHz, CDCl₃): δ_{H} 8.56 ppm (1H, d, J = 5.7 Hz), 7.74 (1H, d, J = 8.2 Hz), 7.68 (1H, t, J = 7.8 Hz), 7.61 (1 H, d, J = 7.8 Hz), 7.46 (1H, d, J = 8.2 Hz), 7.08 (2H, m), 7.03 (1H, s), 6.93 (1H, t, J = 7.6 Hz), 1.66 (15 H, s).

**FKB11: FKB11** was synthesised following the preparation described for compound **FKB10** with the following modifications. Ir dimmer [(Cp*)IrCl₂]₂ (102 mg, 0.128 mmol), 2-(2-pyrindyl)-*1H*-Indole (50 mg, 0.257 mmol). The product was obtained as a yellow powder. Yield: 64.5 mg (27.2 %). HRMS-ESI+: calculated (M-Cl)⁺ 521.1569 m/z, found 521.1564 m/z. 1H NMR (400MHz, CDCl₃): δ_{H} 8.55 ppm (1H, d, J = 5.6 Hz), 7.80 (1H, d, J = 8.3 Hz), 7.67 (1H, t, J = 7.8 Hz), 7.59 (1 H, d, J = 7.8 Hz), 7.44 (1H, d, J = 8.5 Hz), 7.05 (2H, m), 6.94 (1H, m), 1.66 (15 H, s).

**Stability of FKB3, FKB10** and **FKB11:** Each complex was dissolved in MeOD (2.2 mM) and diluted to a final concentration of 1.1 mM with either MeOD or D₂O. ¹H NMR spectra was recorded at t = <10 min, 12 h and 24 h.

Each complex was also dissolved in MeOD (2.2 mM) and diluted to a final concentration of 1.1 mM with D₂O. The sample was then reacted with 1 mol. equiv. of silver nitrate to obtain the fully hydrolyzed specie.

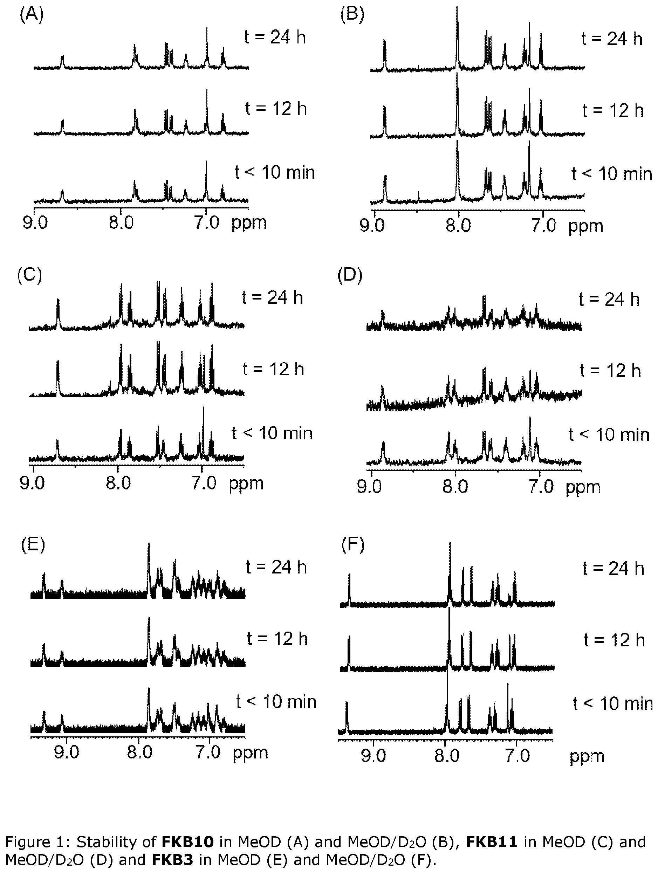

From the NMR spectra of Figure 1 it can be observed that **FKB10** is stable over 24 h both in methanol and in aqueous solution. Furthermore, addition of silver nitrate does not produce any shift of the ¹H NMR resonances, suggesting that ligand substitution occurs replacing the chloride ligand by either methanol or water.

The iridium compound **FKB11** shows no changes in the ¹H NMR spectra, which indicates stability; as a side note, the compound is quite insoluble, and it precipitates over time. The ruthenium complex **FKB3** is fully stable. Interestingly, when the compound is dissolved in 100 % MeOD, two species can be observed, corresponding to the chloride and the methoxide adducts.

Figure 1: Stability of **FKB10** in MeOD (A) and MeOD/D₂O (B), **FKB11** in MeOD (C) and MeOD/D₂O (D) and **FKB3** in MeOD (E) and MeOD/D₂O (F).

### 1.2. Chemosensitivity assays against human cell lines

In vitro chemosensitivity tests were performed against HCT116 (colorectal cancer cell line), A2780 and A2780cisR (cisplatin sensitive and cisplatin resistant ovarian cancer cell lines, respectively). Cancer cell lines were routinely maintained as monolayer cultures in RPMI medium supplemented with 10% foetal calf serum, penicillin (100 I.U./ml) and streptomycin (100 µg/ml), sodium pyruvate (1 mM) and L-glutamine (2 mM). For chemosensitivity studies, cells were incubated in 96-well plates at a concentration of 7.5 × 10³ cells per well and the plates were incubated for 24 hours at 37 °C and a 5% CO2 humidified atmosphere prior to drug exposure.

Complexes were dissolved in dimethylsulfoxide (DMSO) to provide stock solutions which were further diluted with media to provide a range of final concentrations. Drug-media solutions were added to cells (the final concentration of DMSO was less than 0.5% (v/v) in all cases) and incubated for 24 hours at 37 °C and 5% CO₂ humidified atmosphere. The drug-media was removed from the wells and the cells were washed with PBS (100 µL, twice), and 200 µL of complete fresh media were added to each well. The plates were further incubated for 72 hours at 37 °C in a humidified atmosphere of 5% CO2 to allow for a period of recovery. 3-(4,5-dimethylthiazol-2-yl)-2,5-diphenyltetrazolium bromide (MTT) (40 µL, 2.5 mg/mL) was added to each well and incubated for 2 hours at 37 °C and 5% CO2 humidified atmosphere. All solutions were then removed and 100 µL of DMSO was added to each well in order to dissolve the purple formazan crystals. A Thermo Scientific Multiskan EX microplate photometer was used to measure the absorbance in each well at 570 nm. Cell survival was determined as the absorbance of treated cells divided by the absorbance of controls and expressed as a percentage. The IC₅₀ values were determined from plots of % survival against drug concentration. Each experiment was repeated in duplicate of triplicates and a mean value was obtained and stated as IC₅₀ (µM) ± SD. Cisplatin was used as a positive control. *As a side note, the higher the IC₅₀ values are, the less cytotoxic the compounds are.*

**Table 1. IC₅₀ values/ µM ± SD for cisplatin and compounds FKB3, FKB10, and FKB11 against HCT116, A2780, and A2780 cell lines.**

| | | IC₅₀ / µM ± SD | | |
|---|---|---|---|---|
| Complex | Metal | HCT116 | A2780 | A2780cisR |
| **FKB3** | Ru | 107.9 ± 11.5 | 45.4 ± 6.9 | 71.9 ± 11.7 |
| **FKB10** | Rh | 104.2 ± 9.8 | 23.5 ± 1.3 | 41.0 ± 4.7 |
| **FKB11** | Ir | 64.81 ± 4.6 | 22.2 ± 2.1 | 32.6 ± 1.7 |
| **cisplatin** | Pt | 59.8 ± 7.5 | 5.7 ± 0.4 | 11.9 ± 2.2 |

### 2. Antimicrobial activity

### 2.1 Methods

### Growth of organisms

All *Mycobacterium abscessus* (*M. abs*) cultures were grown in Middlebrook 7H9 media, supplemented with glycerol (0.5% v/v), ADC (acid-dextrose-glucose) (10% v/v) and tween80 (0.05% w/v), at 30 °C with shaking at 180 rpm for 72 h. The optical density (OD_{600 nm}) was adjusted to 0.1 prior to microtiter plate set-up.

The gram negative organisms selected were *Escherichia coli* (*E. coli* ATCC, J53 2138E, J53 2140E and I496 ESBL), *Pseudomonas aeruginosa* ATCC and *Klebsiella pneumoniae* H467. All organisms were grown in nutrient broth at 37 °C with 180 rpm shaking, for 18 h. The optical density (OD_{600 nm}) was adjusted to 0.1 prior to microtiter plate set-up.

M.abscessus is an example of a mycobacterium capable of causing lung infections. J53 2138 E, J532140 E and I496ESBL are β-lactamase producing strains. H467 produces a carbapenumase.

### Compounds

Each compound was made in DMSO (dimethyl sulfoxide) to a stock concentration of 100 mM. A master plate was used to generate the desired concentrations for testing. This was achieved by serial 5-fold dilution (repeated 5 times, the final row was DMSO only (0 mM)).

### Experimental Set-up

For each compound, 95 µl of culture (OD 0.1) was added to the microtiter plate and 5 µl of serially diluted compound added. The concentrations tested were: 1 mM, 200 µM, 40 µM, 8 µM, 1.6 µM and 320 nM. All plates were read in the plate reader at 570 nm (T=0). For *M. abs* the plates were incubated at 30 °C for 96 h with reads being taken every 24 h. For gram negative organisms the plates were incubated at 37 °C and read at 0 h and 24 h. After the final read for all organisms the experiment was plated out to observe bactericidal activity. *M. abs* was plated onto Middlebrook 7H11 agar supplemented with glycerol (0.5%) and incubated at 30 °C for 72-96 h. The gram negative organisms were plated onto nutrient agar and incubated at 37 °C for 24 h. Colony forming units were counted for all organisms.

### Data Analysis

All OD readings were transferred to excel and adjusted to account for OD of the broth and compounds. The data was then transferred to GraphPad Prism 8 for data analysis.

### 2.2 Results

Out of the compounds tested, activity was observed for a total of 5 compounds. Three of the compounds, FKB3, FKB10 and FKB11, exhibited varying levels of growth inhibition against all organisms tested.

### M. abscessus ATCC

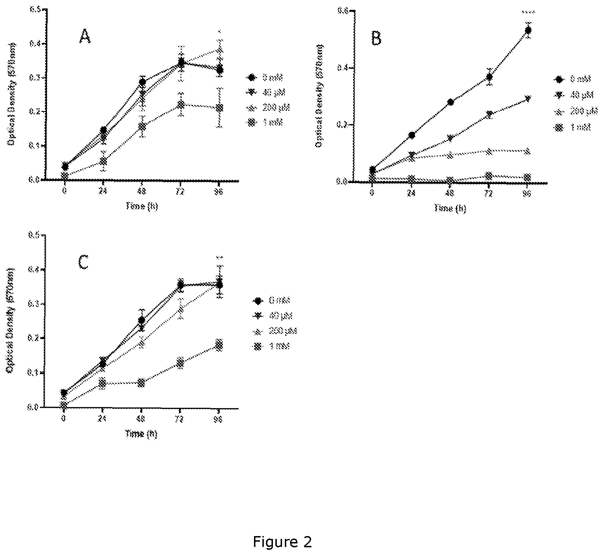

Reduction in optical density reading was observed for all compounds tested against M. *abs* at 1 mM (Figure 2). Uniquely, FKB10 showed a further reduction in optical density at both 200 µM and 40 µM, suggesting the highest potency against *M. abs* (Figure 2B).

### E. coli ATCC

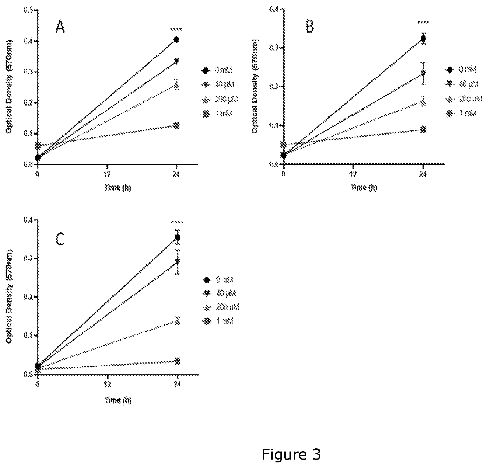

All compounds tested against E. coli ATCC showed activity down to 40 µM (Figure 3). Both FKB10 and FKB11 were the most potent, showing the least change in optical density over 24 h at 1 mM concentration (Figure 3B/C). FKB3 and FKB11 both have minimum bactericidal concentrations (MBC) of 1 mM.

### E. coli J53 2138

All of compounds tested against *E. coli* J53 2138E showed a reduction in optical density.. FKB3 and FKB11 were the only compounds with an observable MBC of 1 mM respectively.

### E. coli J53 2140E

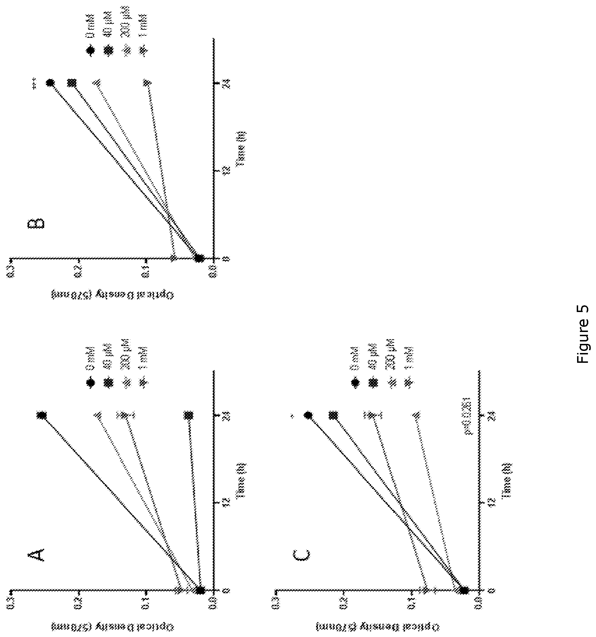

All of the compounds tested show a reduction of optical density over 24 h, with the largest reduction in change of optical density seen for FKB11 at 200 µM (Figure 5C). FKB3 and FKB11 were the only compounds with an observable MBC of 1 mM respectively.

### E. coli 1496 ESBL

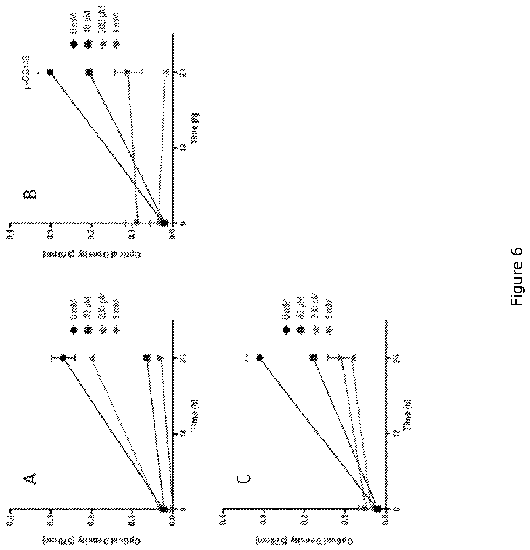

All of the compounds tested show a reduction of optical density over 24 h, with the largest reduction in change of optical density seen for FKB3 at 1 mM, as well as FKB10 at 200 µM (Figure 6A/B). FKB3 and FKB11 were the only compounds with an observable MBC of 1 mM respectively.

### P. aeruginosa ATCC

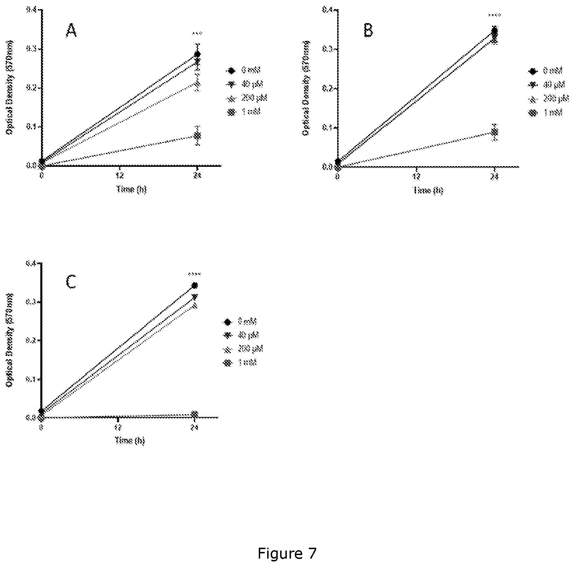

All of the compounds tested caused a reduction in change of optical density at 1 mM (Figure 7). The largest of these reductions occurred when *P. aeruginosa* was treated with FKB11 (Figure 7C). However, despite this, there was no observable MBC for any compound against *P. aeruginosa,* suggesting these compounds are bacteriostatic, rather than bactericidal against this organism.

### K. pneumoniae H467

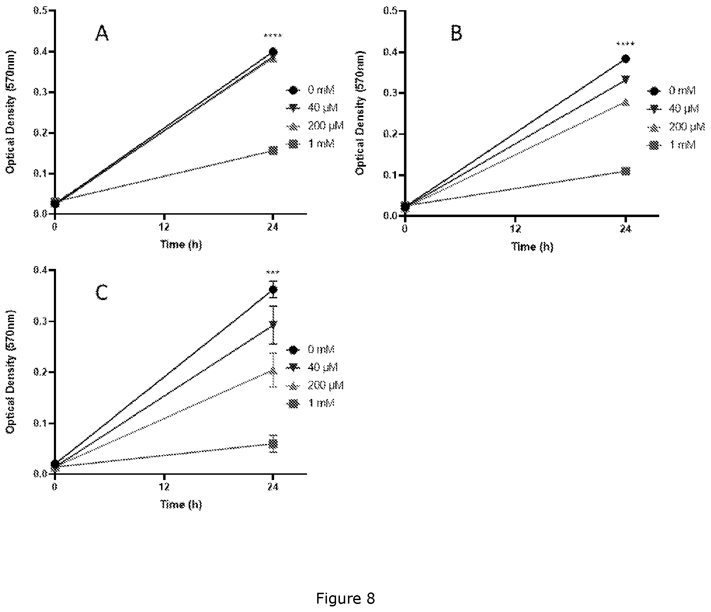

Each compound tested against K. pneumoniae H467 showed a reduction in optical density over 24 h. The largest reduction occurred with the addition of FKB11 at 1 mM (Figure 8C). Both FKB3 and FKB11 gave an MBC of 1 mM.

### Conclusions

These compounds show activity against a wide range of bacterial species, including highly antimicrobial Gram negative strains. Although the optimal concentration for activity is high (1 mM), a number of these compounds are bactericidal against the majority of the organisms screened. Therefore, these compounds represent an intriguing avenue for use and future optimisation and drug development.

## Claims

1. A compound of Formula I for use in the treatment or in the prophylaxis of a bacterial infection: 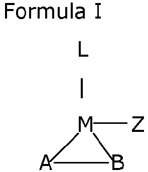
wherein L is a substituted or non-substituted aromatic ligand; Z is a halogen or coordinating ligand; M is a group 8 or group 9 transition metal selected from; Fe, Ru, Co, Rh and Os; wherein one of A or B is a substituted or non-substituted, especially non-substituted, indole; and the second of B or A is a substituted or non-substituted, especially a non-substituted, pyridine,
and A and B coordinate to the transition metal M through the nitrogen atom of the indole or pyridine.

2. A compound of Formula I for use in the treatment or prophylaxis of a bacterial infection according to claim 1 , wherein L comprises 1 or 2 aromatic rings.

3. A compound of Formula I for use in the treatment or prophylaxis of a bacterial infection according to claim 1, wherein L is a substituted or non-substituted cyclopentadienyl ring or a substituted or non-substituted benzene.

4. A compound of Formula I for use in the treatment or prophylaxis of a bacterial infection according to claims 1 to 3, wherein L is substituted by one or more side groups comprising a sugar, hydroxyl, halogen, carboxylate, triazole, amide or a C₁ to C₄ straight or branched chain alkyl group.

5. A compound of Formula I for use in the treatment or prophylaxis of a bacterial infection according to claims 1 to 3 wherein L comprises a C₅ or C₆ aromatic ring.

6. A compound of Formula I for use in the treatment or prophylaxis of a bacterial infection according to claims 1 to 5, wherein L is selected from para-cymene, pentamethylcyclopentadienyl, hexamethyl benzene, biphenyl and cyclopentadienyl.

7. A compound of Formula I for use in the treatment or prophylaxis of a bacterial infection according to claims 1 to 6, wherein Z is selected from Cl, Br, I, water, methanol, dimethyl sulfoxide, acetonitrile and dimethylformamide.

8. A compound of Formula I for use in the treatment or prophylaxis of a bacterial infection according to claims 1 to 7, selected from FKB3, FKB10 and FKB11

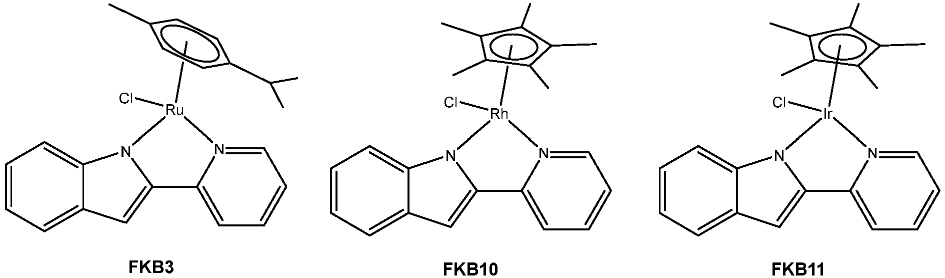

9. A compound of Formula I for use in the treatment or prophylaxis of a bacterial infection according to claims 1-8 wherein the bacterial infection is caused by a Mycobacterium, a Gram-positive bacterium or a Gram-negative bacterium.

10. A compound of Formula I for use in the treatment or prophylaxis of a bacterial infection according to claims 1-9 wherein the bacterium is antibiotic resistant., preferably wherein the bacterium produces a β-lactamase or carbapenumase.

11. A compound of Formula I for use in the treatment or prophylaxis of a bacterial infection of claims 1 to 10, wherein the bacterium is from the genus *Mycobacterium, Escherichia, Salmonella, Clostridium, Streptococcus, Staphylococcus, Pseudomonas, Klebsiella, Acinetobacter* and *Enterobacter,* preferably wherein the bacterium is Mycobacterium absessus, or Escherichia coli, Pseudomonas aeruginosa, or Klebsiella pneumonia.

## Patentansprüche

1. Verbindung der Formel I zur Verwendung bei der Behandlung oder Prophylaxe einer bakteriellen Infektion: 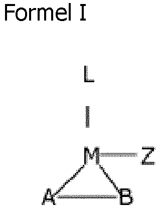 worin L ein substituierter oder unsubstituierter aromatischer Ligand ist, Z ein Halogen oder ein koordinierender Ligand ist, M ein Übergangsmetall der Gruppe 8 oder 9 ist, ausgewählt aus Fe, Ru, Co, Rh und Os, worin eines von A oder B ein substituiertes oder unsubstituiertes, insbesondere unsubstituiertes Indol ist, und das zweite von B oder A ein substituiertes oder unsubstituiertes, insbesondere unsubstituiertes Pyridin ist,
- und A und B über das Stickstoffatom des Indols oder Pyridins an das Übergangsmetall M koordinieren.

2. Verbindung der Formel I zur Verwendung bei der Behandlung oder Prophylaxe einer bakteriellen Infektion gemäß Anspruch 1, wobei L 1 oder 2 aromatische Ringe umfasst.

3. Verbindung der Formel I zur Verwendung bei der Behandlung oder Prophylaxe einer bakteriellen Infektion gemäß Anspruch 1, wobei L ein substituierter oder unsubstituierter Cyclopentadienylring oder ein substituiertes oder unsubstituiertes Benzol ist.

4. Verbindung der Formel I zur Verwendung bei der Behandlung oder Prophylaxe einer bakteriellen Infektion gemäß den Ansprüchen 1 bis 3, wobei L durch eine oder mehrere Seitengruppen substituiert ist, die einen Zucker, Hydroxyl, Halogen, Carboxylat, Triazol, Amid oder eine gerad- oder verzweigtkettige C₁- bis C₄-Alkylgruppe umfassen.

5. Verbindung der Formel I zur Verwendung bei der Behandlung oder Prophylaxe einer bakteriellen Infektion gemäß den Ansprüchen 1 bis 3, wobei L einen aromatischen C₅- oder C₆-Ring umfasst.

6. Verbindung der Formel I zur Verwendung bei der Behandlung oder Prophylaxe einer bakteriellen Infektion gemäß den Ansprüchen 1 bis 5, wobei L ausgewählt ist aus p-Cymol, Pentamethylcyclopentadienyl, Hexamethylbenzol, Biphenyl und Cyclopentadienyl.

7. Verbindung der Formel I zur Verwendung bei der Behandlung oder Prophylaxe einer bakteriellen Infektion gemäß den Ansprüchen 1 bis 6, wobei Z aus Cl, Br, I, Wasser, Methanol, Dimethylsulfoxid, Acetonitril und Dimethylformamid ausgewählt ist.

8. Verbindung der Formel I zur Verwendung bei der Behandlung oder Prophylaxe einer bakteriellen Infektion gemäß den Ansprüchen 1 bis 7, ausgewählt aus FKB3, FKB10 und FKB11

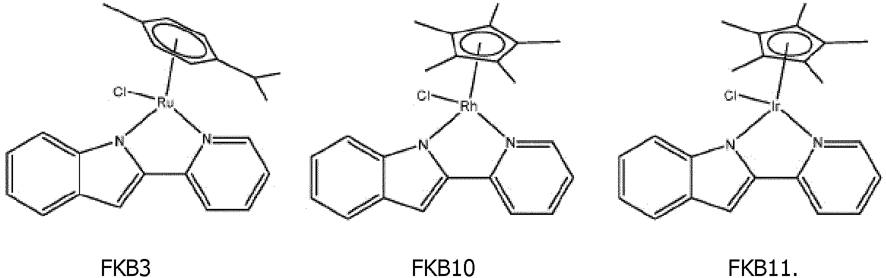

9. Verbindung der Formel I zur Verwendung bei der Behandlung oder Prophylaxe einer bakteriellen Infektion gemäß den Ansprüchen 1 bis 8, wobei die bakterielle Infektion durch ein Mykobakterium, ein grampositives Bakterium oder ein gramnegatives Bakterium verursacht ist.

10. Verbindung der Formel I zur Verwendung bei der Behandlung oder Prophylaxe einer bakteriellen Infektion gemäß den Ansprüchen 1 bis 9, wobei das Bakterium antibiotikaresistent ist, vorzugsweise wobei das Bakterium eine β-Lactamase oder Carbapenumase produziert.

11. Verbindung der Formel I zur Verwendung bei der Behandlung oder Prophylaxe einer bakteriellen Infektion nach einem der Ansprüche 1 bis 10, wobei das Bakterium aus der Gattung *Mycobacterium, Escherichia, Salmonella, Clostridium, Streptococcus, Staphylococcus, Pseudomonas, Klebsiella, Acinetobacter* und *Enterobacter* stammt, vorzugsweise wobei das Bakterium Mycobacterium absessus oder Escherichia coli, Pseudomonas aeruginosa oder Klebsiella pneumonia ist.

## Revendications

1. Un composé de formule I à utiliser dans le traitement ou la prophylaxie d'une infection bactérienne : 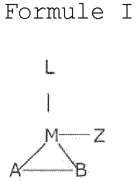
dans laquelle L est un ligand aromatique substitué ou non substitué ; Z est un halogène ou un ligand de coordination ; M est un métal de transition du groupe 8 ou du groupe 9 choisi parmi ; Fe, Ru, Co, Rh et Os ; dans laquelle l'un de A ou B est un indole substitué ou non substitué, spécialement non substitué ; et le second de B ou A est une pyridine substituée ou non substituée, spécialement non substituée,
et A et B font la coordination au métal M de transition par l'atome d'azote de l'indole ou de la pyridine.

2. Un composé de formule I à utiliser dans le traitement ou la prophylaxie d'une infection bactérienne suivant la revendication 1, dans lequel L comprend ou 1 ou 2 noyaux aromatiques.

3. Un composé de formule I à utiliser dans le traitement ou la prophylaxie d'une infection bactérienne suivant la revendication 1, dans lequel L est un noyau cyclopentadiènyle substitué ou non substitué ou un benzène substitué ou non substitué.

4. Un composé de formule I à utiliser dans le traitement ou la prophylaxie d'une infection bactérienne suivant les revendications 1 à 3, dans lequel L est substitué par un ou plusieurs groupes latéraux comprenant un sucre, hydroxyle, halogène, carboxylate, triazole, amide ou un groupe alcoyle à chaîne linéaire ou ramifiée en C₁ à C₄.

5. Un composé de formule I à utiliser dans le traitement ou la prophylaxie d'une infection bactérienne suivant les revendications 1 à 3, dans lequel L comprend un noyau aromatique en C₅ ou en C₆.

6. Un composé de formule I à utiliser dans le traitement ou la prophylaxie d'une infection bactérienne suivant les revendications 1 à 5, dans lequel L est choisi parmi paracymène, pentaméthylcyclopentadiènyle, hexaméthylbenzène, biphényle et cyclopentadiènyle.

7. Un composé de formule I à utiliser dans le traitement ou la prophylaxie d'une infection bactérienne suivant les revendications 1 à 6, dans lequel Z est choisi parmi Cl, Br, I, eau, méthanol, diméthyl sulfoxyde, acétonitrile et diméthylformamide.

8. Un composé de formule I à utiliser dans le traitement ou la prophylaxie d'une infection bactérienne suivant les revendications 1 à 7, choisi parmi FKB3, FKB10 et FKB11

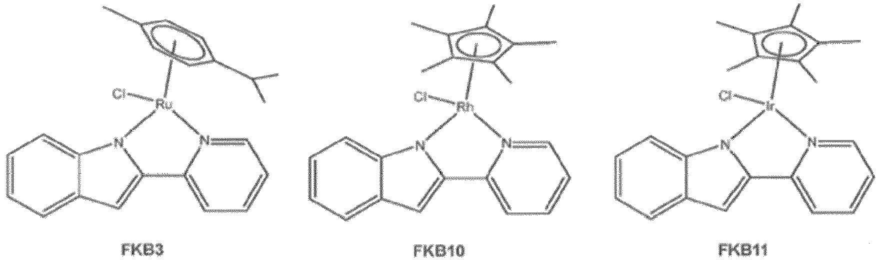

9. Un composé de formule I à utiliser dans le traitement ou la prophylaxie d'une infection bactérienne suivant les revendications 1 à 8, dans lequel l'infection bactérienne est provoquée par une mycobactérie, une bactérie prenant le Gram ou une bactérie ne prenant pas le Gram.

10. Un composé de formule I à utiliser dans le traitement ou la prophylaxie d'une infection bactérienne suivant l'une des revendications 1 à 9, dans lequel la bactérie est résistante aux antibiotiques, dans lequel de préférence la bactérie produit une β-lactamase ou une carbapénumase.

11. Un composé de formule I à utiliser dans le traitement ou la prophylaxie d'une infection bactérienne suivant les revendications 1 à 10, dans lequel la bactérie est du genre *mycobactérium, Eschérichia, Salmonella, Clostridium, Streptococcus, Staphylococcus, Pseudomonas, Klebsiella, Acinétobacter* et *Entérobacter,* dans lequel de préférence la bactérie est Mycobactérium absessus, ou Eschérichia coli, Pseudomonas aeruginosa ou Klebsiella pneumonia.
